# DEMANDE DE BREVET EUROPEEN

(11) **EP 0 856 308 A2**
(43) Date de publication de la demande: **05.08.1998**
(21) Numéro de dépôt: 98870020.9
(22) Date de dépôt: 03.02.1998
(51) Int. Cl.: A61K 7/15, A61K 7/48

(54) **Composition de pré-rasage**

(30) Priorité: 03.02.1997 BE 9700098
(71) Demandeur: Iacolino, Antonio, 4400 Flémalle (BE)
(72) Inventeur: Iacolino, Antonio, 4400 Flémalle (BE)
(74) Mandataire: Colens, Alain

(57) **Abrégé**

L'invention concerne une composition de pré-rasage obtenue par mélange d'un hydrogel et d'une crème huile dans eau formant une substance semi-crémeuse hydratante, non moussante. Le composante crème est de préférence à base de polysorbate et la composante gel à base de carbopol.

## Description

La présente invention concerne une composition cosmétique et son utilisation.

Plus particulièrement, l'invention se rapporte à une composition de pré-rasage destinée à être appliquée sur la partie de la peau du visage qui doit être rasée avec un rasoir à lame manuel (rasoir mécanique à lame amovible, jetable ou non).

Ce type de rasage s'effectue normalement en appliquant préalablement une mousse de rasage qui permet de lubrifier la surface de contact et de faciliter ainsi la coupe. Cette mousse agit également comme traceur ou indicateur, l'utilisateur pouvant visuellement apprécier les surfaces déjà traitées par le rasoir. Les mousses actuelles sont souvent contenues dans des récipients sous pression (bonbonnes) ce qui conduit à certaines limitations quant à leurs formulations.

Les utilisateurs sont cependant fréquemment confrontés à des irritations de la peau.

Selon l'invention, on a constaté que celles-ci sont le plus souvent dues au fait que la surface de la peau, malgré l'effet de lubrification de la mousse, n'est pas suffisamment humidifiée. Le rasage a tendance à s'effectuer alors "à sec".

En effet, les mousses traditionnelles n'ont pas pour effet d'hydrater réellement la surface de la peau. Elles restent au dessus de la peau et laissent la peau pratiquement sèche.

On a cependant proposé des gels à raser contenant des agents lubrifiants qui ne sont pas à base de savon et ne produisent pas de mousse.

Comme les mousses cependant, ces gels ont tendance à encombrer la lame ce qui rend le nettoyage de celle-ci à l'eau plus fréquent et augmente l'usure, donc le nombre de fois qu'une même lame peut être utilisée.

L'invention propose donc une composition à appliquer sur la peau, avant l'application éventuelle de la mousse, et qui permet de maintenir la surface de la peau humide durant toute l'opération de rasage.

Le rasage est plus rapide, plus facile et moins irritant. A l'usage le produit s'avère aussi plus économique étant donné une utilisation moindre de mousse à raser. La durée de vie des lames est par ailleurs significativement augmentée et le nettoyage à l'eau de celles-ci est moins long et plus facile.

La préparation selon l'invention tient sur la peau en y formant une couche mince qui retient l'eau qu'on y applique permettant ainsi l'hydratation stable en surface. Cette couche n'est pas éliminée par le passage de la lame du rasoir.

Le produit est une préparation semi-solide, subjectivement à mi chemin entre un gel et une crème.

La composition selon l'invention se caractérise par la présente d'ingrédients utilisés classiquement pour la formation de gels et d'ingrédients utilisés classiquement dans les compositions de crèmes (plus pénétrantes dans la peau), en l'absence de savon.

La composante "gel" sera de préférence un hydrogel synthétique ou semi-synthétique. Il sera de préférence à base de carbopol (carboxypolyméthylène), plus particulièrement de carbopol 940 éventuellement de préférence associé à une base telle la triéthanolamine (p.e. à 125 % en poids de carbopol). La concentration de carbopol varie de 0,1 à 4%, de préférence de 0,2 à 2%.

D'autres dérivés des polymères de l'acide acrylique peuvent être utilisés, neutralisé par NaOH ou NH₄OH, la triéthanolamine, la diisopropylamine, la triéthylamine ou la triamylamine.

Encore d'autres catégories d'hydrogels peuvent être utilisées , par exemple
- les celluloses et dérivés tels que la cellulose microcristalline (Avicel) utilisée souvent en association avec d'autres agents viscosifiants tels que carboxyméthylcelluloses ou méthylcelluloses. Ces derniers peuvent être utilisés séparément comme composante hydrogel.
- des silicates tels
   laponite silicate de magnésium synthétique
   bentonite silicate d'alumine
   Veegum silicate complexe de magnésium et aluminium
- gélifiants des solvants et des corps gras :
   aérosil acide silicique de très grande finesse
   bentones type de montmorillonites avec radicaux alkylammonium
   alugels stéarate d'aluminium

Le reste est constitué d'ingrédients susceptibles de former une crème avec éventuellement des excipients additionnels.

La composante "crème" est de préférence une crème "huile dans eau" , à base de polysorbates, par exemple associés à un alcool tel l'alcool cétylique. Ces crèmes sont rafraîchissantes, facilement lavables et se fixent facilement à la peau. Utilisées isolément elles se déssèchent facilement.

Le polysorbate, de préférence le polysorbate 80, se trouve dans des concentrations variant de 1 à 15%, de préférence de 2 à 10%. On adjoint avantageusement de 1 à 7% de vaseline. Dans la phase aqueuse on ajoute comme agent hydratant 1 à 15 % de sorbitol.

La composante crème peut comprendre des facteurs de consistance tels des alcools gras (alcool myristique, cétylique, stéarylique, cétyl stéarylique) à des concentration variant de 0,2 à 10%. Ces alcools gras peuvent être utilisés seuls ou en association avec des agents émulsifiants tel que cétomagropol, ou d'autres esters polyglycoliques pour donner des cires autoémulsives (émulcires, émulgades, tansactols etc..)

Le produit final comporte de 65 à 90% d'eau, de préférence de 75 à 85% d'eau, par exemple environ 80% d'eau.

Selon un mode de réalisation actuellement préféré la composition de pré-rasage selon l'invention comprendra 1 à 3 % en poids d'alcool cétylique, 0,2 à 2 % de carbopol, 3 à 8 % de Polysorbate-80 (Tween), 0,1 à 1,5 % de triéthanolamine, 1 à 7 % de vaseline, 2 à 8 % de propylène glycol, le restant étant constitué d'eau.

Des agents stabilisants, conservants et d'autres excipients ou additifs bien connus peuvent être ajoutés. On citera par exemple l'E.D.TA., la nipagine, le nipazole, le sorbitol. De manière classique, on peut ajouter également des parfums et/ou des colorants et/ou des agents absorbeurs d'UV. On peut ajouter en particulier un parfum hypoallergénique, sans alcool si l'on désire éviter des allergies pour un plus grand nombre de personnes possibles.

L'invention concerne également une méthode de rasage comprenant les étapes suivantes :
- application d'une composition hydratante de surface, en particulier telle que décrite dans le présent exposé
- application d'eau
- application d'une mousse de rasage
- coupe au rasoir
- rincage de la mousse et de la crème à l'eau.

Le rincage permet d'éliminer tous les produits préalablement appliquer en ne laissant aucun résidu gras ou collant.

Exemples non-limitatifs de compositions selon l'invention

### Exemple 1

- alcool cétylique 2 g 1-hexadécanol émollient
- carbopol 940 0,75 g carboxypolyméthylène (ionique)
- E.D.T.A. 20 mg agent chélateur
- Nipagine 20 mg methyl para hydroxybenzoate
- Nipazol 80 mg propyl para hydroxybenzoate
- Polysorbate 80 5,8 g (Tween)
- Propylène glycol 5,6 g
- Sorbitol 1,75 g
- Triéthanolamine 0,94 g
- Vaseline 2,95 g
- Eau distillée ad 100 g

### Exemple 2

| | |
|---|---|
| Alcool cétylstéarilique | 0,28 g |
| cetometacrogol | 0,07 g |
| cetiol | 0,7 g |
| carbopol | 0,75 g |
| E.DT.A | 20 mg |
| nipagine | 20 mg |
| nipazol | 80 mg |
| polysorbate 80 | 5 g |
| propylène glycol | 5,4 g |
| triéthanolamine | 0,96 g |
| eau distillée ad | 100 g |

### Exemple 3

| | |
|---|---|
| Alcool cétylique | 1,8 g |
| carbopol 940 | 1,4 g |
| Cire blanche | 0,15 g |
| E.DT.A | 35 mg |
| laurylsulfate de sodium | 3,3 g |
| nipagine | 20 mg |
| nipazol | 80 mg |
| polysorbate 80 | 4 g |
| propylène glycol | 11,5 g |
| triéthanolamine | 1,7 g |
| eau distillée ad | 100 g |

### Exemple 4

| | |
|---|---|
| Alcool cétylique | 1,15 g |
| carbopol 940 | 0,44 g |
| E.DT.A | 12 mg |
| eucérine | 4,5 g |
| nipagine | 20 mg |
| nipazol | 80 mg |
| polysorbate 80 | 12 g |
| propylène glycol | 5 g |
| triéthanolamine | 0,55 g |
| vaseline | 1,7 g |
| eau distillée ad | 100 g |

### Exemple 5

| | |
|---|---|
| Alcool cétylique | 1,2 g |
| carbopol 940 | 0,75 g |
| E.D.T.A | 20 mg |
| eucérine | 1,75 g |
| nipagine | 20 mg |
| nipazol | 80 mg |
| polysorbate 80 | 3,3 g |
| propylène glycol | 5,55 g |
| sorbitol | 1,05 g |
| triéthanolamine | 0,92 g |
| vaseline | 1,75 g |
| eau distillée ad | 100 g |

### Mode opératoire général pour une composition comprenant les ingrédients des exemples susmentionnés.

a) dissoudre de la nipagine et du nipazol à chaud dans le propylène glycol
b) triturer soigneusement le carbopol 940 dans la solution de propylène glycol ainsi préparée (I)
c) dissoudre l'EDTA dans une partie d'eau et mélanger avec la triéthanolamine (II)
d) ajouter (II) à (I) par petites quantités et mélanger pour obtenir un gel uniforme (III). On laisse reposer pendant 24 heures.
e) chauffer la vaseline, l'alcool cétylique ou cétylstéarylique et le polysorbate 80 jusqu'à obtention d'une solution d'une part, et d'autre part porter l'autre partie d'eau additionnée du sorbitol à la même température, mélanger les deux solutions pour obtenir une crème (IV)
f) mélanger le gel obtenu en d) avec la crème obtenue en e) pour produire le produit selon l'invention.

Selon un autre aspect de l'invention on propose un conteneur à deux compartiments et deux têtes de distribution adjacentes, par exemple du type poussoir, pouvant délivrer ainsi, successivement, la composition semi-crémeuse selon l'invention et une mousse à raser classique. Une bonbonne peut ainsi comprendre deux compartements semi-cylindriques longitudinaux comportant à une extrémités les têtes de distribution à poussoir pouvant délivrer, de préférence successivement, la composition selon l'invention et la mousse de rasage.

Selon encore un autre aspect de l'invention, on propose une trousse de rasage comprenant au moins un récipient avec la composition selon l'invention et un récipient comportant la mousse de rasage, éventuellement accompagné d'un rasoir et/ou d'autres ustensiles.

En résumé, l'invention propose une composition "crémo-gel" se fixant facilement à la peau et susceptible d'y rester même après apport d'une hydratation maximale. Plus particulièrement, cette hydratation persiste durant tout le temps d'un rasage, avec addition éventuelle de mousse. L'hydratation est renforcée par l'effet d'une grande proportion d'un agent émulsifiant, p.e. le polysorbate, constituant en grande partie la phase huileuse de la partie crème, ce qui permet un passage rapide, aisé et très peu irritant. Le crémo-gel de par sa composition et la présence d'une proportion significative d'un agent émulsifiant se détache facilement du rasoir, rendant le rasage plus aisé, et permet un lavage rapide et efficace de la peau en fin d'opération.

## Revendications

1. Composition de pré-rasage caractérisée en ce qu'elle est constitué d'une substance semi-crémeuse hydratante, constituée de plus de 60% d'eau, essentiellement non moussante, à appliquer sur la peau du visage.

2. Composition de prérasage caractérisée en ce qu'elle peut être obtenue par mélange d'un hydrogel et d'une crème huile dans eau.

3. Composition selon la revendication 1 ou 2 caractérisée en ce qu'elle comprend un polysorbate pour sa composante crème et du carbopol pour sa composante gel.

4. Composition selon n'importe laquelle des revendications précédentes caractérisée en ce que le polysorbate se présente à une concentration pondérale variant de 1 à 15%, de préférence de 2 à 10 %.

5. Composition selon n'importe laquelle des revendications précédentes caractérisée en ce que le carbopol se présente à une concentration pondérale de 0,1 à 4%, de préférence de 0,2 à 2%.

6. Composition selon n'importe laquelle des revendications précédentes caractérisée en ce qu'elle comprend en outre un alcool gras, plus particulièrement de l'alcool cétylique, à une concentration pondérale variant de 0,1 à 15%, de préférence de 0,2 à 10 %.

7. Composition selon n'importe laquelle des revendications précédentes caractérisée ne ce qu'elle comprend en outre du propylène glycol et/ou du sorbitol.

8. Composition selon n'importe laquelle des revendications précédentes caractérisée en ce qu'elle comprend en outre une base tel que le NaOH, le NH4OH, de la triéthanolamine ou une autre base aminée organique.

9. Composition selon n'importe laquelle des revendications précédentes caractérisée en ce qu'elle comprend en outre de la vaseline.

10. Composition selon n'importe laquelle des revendications précédentes caractérisée en ce qu'elle comprend en outre de la nipagine ou du nipazol.

11. Conteneur-distributeur ou trousse comportant un conteneur pour une composition suivant n'importe laquelle des revendications précédentes caractérisé en ce qu'il comprend en outre un second réservoir associé à un dispositif de distribution pour de la mousse à raser.

12. Procédé d'obtention d'une composition selon les revendications 1 à 11 caractérisé en ce qu'on mélange une composition de gel à base de carbopol à une composition crémeuse à base de polysorbate.
